Europäisches Patentamt
European Patent Office
Office européen des brevets

(19)

(11) Publication number: **0 221 719 A2**

# EUROPEAN PATENT APPLICATION

(12)

(21) Application number: 86308148.5

(22) Date of filing: 21.10.86

(51) Int. Cl.[4]: C 07 D 501/36, A 61 K 31/545

(30) Priority: 22.10.85 HU 406485

(43) Date of publication of application: 13.05.87 Bulletin 87/20

(84) Designated Contracting States: AT BE CH DE FR GB IT LI NL SE

(71) Applicant: BIOGAL GYOGYSZERGYAR
Pallagi u. 13.
H-4042 Debrecen (HU)

(72) Inventor: Marossy, Katalin
Mikzzáth Kálmán ut 46
H-4032 Debrecen (HU)

Mihók, Miklós
Szabó I. altábornagy tér 2
H-4032 Debrecen (HU)

Mihók, Ildikó
Szabó I. altábornagy tér 2
H-4032 Debrecen (HU)

Jancsó, Sándor
Kardos u. 28
H-4028 Debrecen (HU)

Elek, Sándor
Német L. u. 25
H-4032 Debrecen (HU)

Koczka, István
Bem József u. 14, IV. emelet 1
1027 Budapest (HU)

Prékopa, Agnes
Simoyi u. 32
H-4028 Drebrecen (HU)

Kiss, Ilona
Monostor u. 12
H-4029 Drebrecen Mihók, Miklós

Huszthy, Erzsébet
Jerikó u. 9
H-4032 Drebrecen Mihók, Ildikó

Soltész, Katalin
Vöröshadsereg u. 1
H-4025 Debrecen Jancsó, Sándor

(74) Representative: Hedley, Nicholas James Matthew et al
T.Z. Gold & Company 9 Staple Inn
London WC1V 7QH (GB)

(54) **Novel cephalosporin derivatives, processes for their preparation and pharmaceutical compositions containing them.**

(57) The invention relates to novel 7β-[2-(2-aminothiazol-4-yl)-(Z)-2-alkoxyimino-acetamido]-3-[(2-mercapto-1,3,4-thiadiazol-5-yl)thiomethyl]-ceph-3-em-4-carboxylic acids of the formula

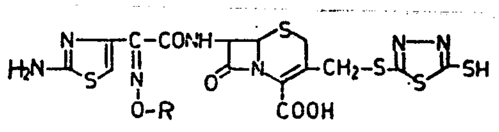

(I) .

wherein R represents a $C_{1-4}$ alkyl group, and pharmaceutically acceptable salts and esters thereof. The antibacterial compounds of the invention can be prepared by methods well known in the cephalosporin chemistry.

EP 0 221 719 A2

## Description

### NOVEL CEPHALOSPORIN DERIVATIVES, PROCESSES FOR THEIR PREPARATION AND PHARMACEUTICAL COMPOSITIONS CONTAINING THEM

The present invention relates to novel 7β-[2-(2-amino-thiazol-4-yl)-(Z)-2-alkoxyimino-acetamido]-3-[(2-mercapto-1,3,4-thiadiazol-5-yl)thiomethyl]-ceph-3-em-4-carboxylic acids of the formula

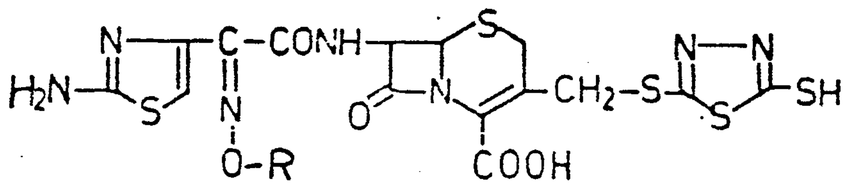

(I)

as well as pharmaceutically acceptable salts and esters thereof.

Several attempts are known from the literature wherein 3,7-disubstituted cephalosporin derivatives containing optionally substituted 5-membered heterocyclic rings in both of these positions were prepared. There are several patent specifications known (see later) which disclose the preparation of derivatives of 7-ACA containing substituted thiazol and thiadiazol rings in positions 3 and 7, respectively. There are also known attempts for the preparation of 7β-[2-(2-aminothiazol-4-yl)-(Z)-2-alkoxyimino-acetamido]-3-[(1,2,3-thiadiazol-5-yl)thiomethyl]-ceph-3-em-4-carboxylic acids and for their testing in respect of the antibacterial activity (J. Antibiot., Vol. XXXVI., No. 2., 179 - 180, 1983).

According to the Belgian patent specification No. 823,861 a 2-aminothiazol ring can be introduced into the 7th position of 7-ACA via an acetylamino group. Some of the compounds prepared according to this patent specification, namely 7-[2-(2-aminothiazol-4-yl)acetamido]-3-[(1,3,4-thiadiazol-2-yl)thiomethyl]-ceph-3-em-4-carboxylic acid, 7-[2-(2-aminothiazol-4-yl)acetamido]-3-[(5-methyl-1,3,4-thiadiazol-2-yl)thiomethyl]-ceph-3-em-4-carboxylic acid and 7-[2-(2-aminothiazol-4-yl)acetamido]-3-[(5-mercapto-1,3,4-thiadiazol-2-yl)thiomethyl]-ceph-3-em-4-carboxylic acid, possess a substantial antibacterial action, similar to that of Cefazolin {7-(1-(1H)-tetrazolylacetamido)-3-[2-(5-methyl-1,3,4-thiadiazolyl)thiomethyl]-ceph-3-em-4-carboxylic acid; The Merck Index, Tenth Edition, 1901.}. An outstanding action could be observed against Proteus vulgaris and Staphylococcus aureus.

The Belgian patent specification No. 889,913 describes 7β-[2-(2-aminothiazol-4-yl)-2-methoxyimino-acetamido]-3-[(substituted 1,2,3-thiadiazol-5-yl)thiomethyl]-ceph-3-em-4-carboxylic acid derivatives containing a $C_{1-6}$ alkyl group as the substituent in position 4 of the thiadiazol moiety. If this alkyl group is a methyl group, the corresponding compound is very active against Gram-negative bacteria in a broad spectrum, while it exerts little activity against Gram-positive bacteria.

The European patent publication No. 0,113,243 described 7 β-[2-(2-aminothiazol-4-yl)-2-methoxyimino-acetamido]-3-[(2-amino-1,3,4-thiadiazol-5-yl)thiomethyl]-ceph-3-em-4-carboxylic acid and its pivaloyloxymethyl ester as pharmacologically active compounds. These compounds possess a better action against E. coli, S. aureus, P. vulgaris, P. aeruginiosa and S.marcescens than Cefotaxime {7-[2-(2-amino-4-thiazolyl)-2-methoxyiminoacetamido]-cephalosporanic acid; The Merck Index, Tenth Edition, 1907.}.

It has now been found that the novel 7β-[2-(2-aminothiazol-4-yl)-(Z)-2-alkoxyimino-acetamido]-3-[(2-mercapto-1,3,4-thiadiazol-5-yl)thiomethyl]-ceph-3-em-4-carboxylic acids of the formula(I), wherein R stands for a $C_{1-4}$ alkyl group, and their pharmaceutically acceptable salts and esters possess outstanding antibacterial properties in comparison with the known and tested analogues.

Thus, in one aspect, the invention relates to compounds of the formula

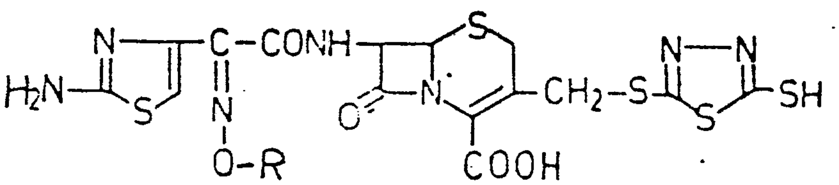

(I)

wherein R stands for a $C_{1-4}$ alkyl group, and their pharmaceutically acceptable salts and esters.

The compounds of the formula (I) are active, first of all, against Gram-negative bacteria. Moreover, it has been found that one of the compounds of the invention, namely 7β-[2-(2-aminothiazol-4-yl)-(Z)-2-methoxyimino-acetamido]-3-[(2-mercapto-1,3,4-thiadiazol-5-yl)thiomethyl]-ceph-3-em-4-carboxylic acid, and its pharmaceutically active salts and esters have an antibacterial action which surpasses the action of the other compounds of the invention. These compounds are rather active against a wide range of Gram-negative bacteria and they possess, in contrast with the analogous cephalosporins, an outstanding action against

Pseudomonas Spp. Said 7β-[2-(2-aminothiazol-4-yl)-(Z)-2-methoxyimino-acetamido]-3-[(2-mercapto-1,3,4-thiadiazol-5-yl)thiomethyl]-ceph-3-em-4-carboxylic acid was subjected to chemotherapeutic tests in the form of its sodium salt. This derivative is referred to under the code number "SZMFO 110" in the tests described hereinbelow.

The MIC values expressed in mcg./ml. of SZMFO 110 after 24 hours are contained in Table 1.

| Bacterium strain tested | MIC (mcg./ml.) |
|---|---|
| Escherichia coli K12 | 0.03 |
| Escherichia coli 6R | 0.25 |
| Escherichia coli poliresistens | 0.16 |
| Escherichia coli R-222 | 0.03 |
| Escherichia coli R-15 | 0.16 |
| Klebsiella sp. ATCC 10031 | 0.007 |
| Proteus vulgaris XL | 0.007 |
| Pseudomonas pyocyanea NTCT 10.490 | 0.31 |
| Salmonella typhi murium | 0.06 |
| Shigella sonnei | 0.06 |
| Bacillus subtilis ATCC 6633 | 0.08 |
| Staphylococcus aureus 1110 | 6.2 |
| Staphylococcus aureus 53 | 1.6 |
| Staphylococcus aureus Smith | 1.25 |
| Staphylococcus epidermidis A CC I-12228 | 0.6 |
| Streptococcus haemolyticus Pneumo | 0.03 |
| Streptococcus haemolyticus A118 | 0.03 |
| Streptococcus haemolyticus Robb | 0.03 |
| Clostridium perfringens Cp. | 0.004 |

The following Table 2 clearly proves that SZMFO 110 is much more active against Pseudomonas aeruginosa than several well-known and widely used third generation cephalosporin derivatives.

Table 2

| Tested compound | MIC (mcg./ml.) |
|---|---|
| SZMO 110 | 0.31 |
| Cefoperazone (see later) | 8.0 |
| Cefotaxime (see earlier) | 32.0 |
| Ceftazimide[1)] | 4.0 |
| Ceftizoxime[2)] | 64.0 |
| Cefmenoxime[3)] | 64.0 |

1) 1-//(6R,7R)-7-/2-(2-amino-4-thiazolyl)glyoxylamido/--2-carboxy-8-oxo-5-thia-1-azabicyclo/4.2.0/oct-2-ene--3-yl/methyl/pyridinium hydroxide inner salt $7^2$-(Z)-/O-(1-carboxy-1-methylethyl)oxime/; The Merck Index, tenth edition, 1913.

2) (6R,7R)-7-/2-(2-amino-4-thiazolyl)glyoxylamido/-8--oxo-5-thia-1-azabicyclo/4.2.0/oct-2-ene-2-carboxylic acid $7^2$-(Z)- (O-methyloxime); The Merck Index, tenth edition, 1915.

3) (6R,7R)-7-/2-(2-amino-4-thiazolyl)glyoxylamido/-3-//(1-methyl-1H-tetrazol-5-yl)thio/methyl/-8-oxo-5-thia--1-azabicyclo/4.2.0/oct-2-ene-2-carboxylic acid $7^2$-(Z)-(O-methyloxime); The Merck Index, tenth edition, 1902.

The effectivity of SZMFO 110 was tested in vivo against Salmonella typhi murium and Proteus vulgaris by using female CFLP mouse as test animals in groups comprising 5 animals weighing 79 to 125 g. per group. The test compounds were administered subcutaneously. As control material a known cephalosporin derivative, i.e. Cefoperazone {7-[D-(-)-α-(4-ethyl-2,3-dioxo-1-piperazinecarboxamido)-α-(4-hydroxyphenyl)-acetamido]--3-[[(1-methyl-1H-tetranol-5-yl)thio]methyl]-ceph-3-em-4-carboxylic acid; The Merck Index, Tenth Edition, 1905.} was used. The results of this test are contained in Table 3.

Table 3

| Tested compound | Dosis mg./kg. | living animal / group | | ED50 (mg./kg.) | |
|---|---|---|---|---|---|
| | | 1st day | 3rd day | 1st day | 3rd day |
| SZMFO 110 | 0.2 | 0 | 0 | 0.62 | 0.62 |
| | 1 | 4 | 4 | | |
| | 5 | 5 | 4 | | |
| | 25 | 5 | 5 | | |
| | 100 | 5 | 4 | | |
| Cefoperazone | 0.2 | 1 | 1 | 0.44 | 3.7 |
| | 1 | 4 | 1 | | |
| | 5 | 5 | 3 | | |
| | 25 | 4 | 4 | | |
| | 100 | 5 | 4 | | |

As regards the data contained in table 3 above, the following explanation is given.

During the tests in question the mice were infected artifically in a way ensuring their death without a treatment. This was achieved by injecting directly the microorganisms used for the infection into the abdomen of the mice. In view of this treatment the animals were dead within 24 hours. When using SZMFO 110 in a dose as low as 1 mg./kg. of body weight practically all of the test animals remained alive. Higher doses are similarly effective. It is highly characteristic that one dose of SZMFO 110 saved the animals, in sharp contrast with Cefoperazone which was able to save finally the test animals only in much higher doses or when used several times. The data in table 3 clearly prove this explanation: the number of the mice kept alive on the 3rd day is different in the case of SZMFO 110 and Cefoperazone. While a fully complete therapeutic effect can be achieved with SZMFO 110 in a dose of 1 mg./kg. of body weight, the same effect can be achieved only in a dose of 25 mg./kg. of the body weight in the case of Cefoperazone.

In the other aspect, the invention relates to a process for the preparation of the compounds of the formula

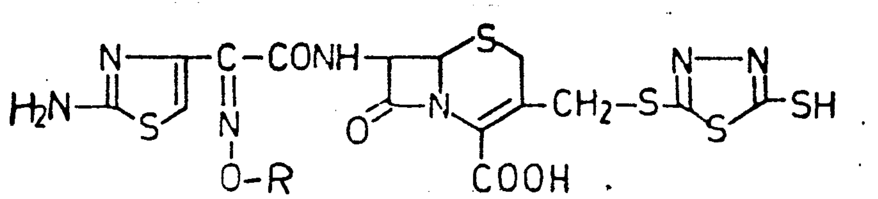

(I),

wherein R stands for $C_{1-4}$ alkyl group, and their pharmaceutically acceptable salts and esters. This process is characterized by

a) acylating a carboxy-protected derivative of the compound of the formula

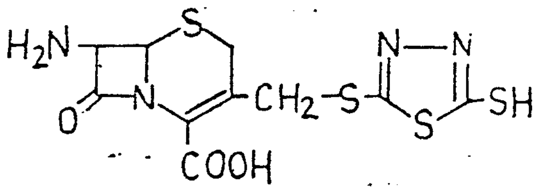

(IV)

with a compound of the formula

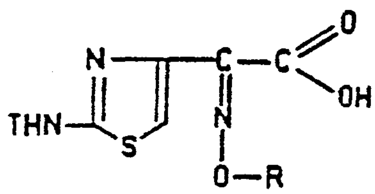

(V),

wherein T is an amino-protecting group and R is as defined above, or a reactive derivative thereof and removing the protecting groups; or

b) reacting a carboxy-protected derivative of the compound of the formula

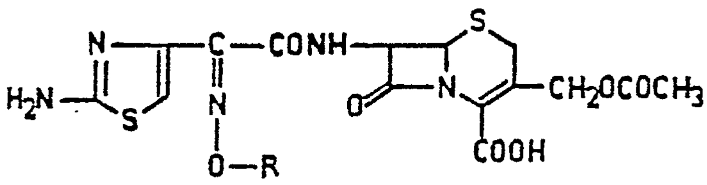

(VII)

with a compound of the formula

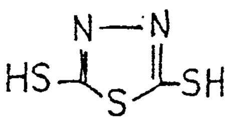

(III),

and removing the protecting group; or

c) removing the T amino-protecting group and, if present, the carboxy-protecting group of a compound of the formula

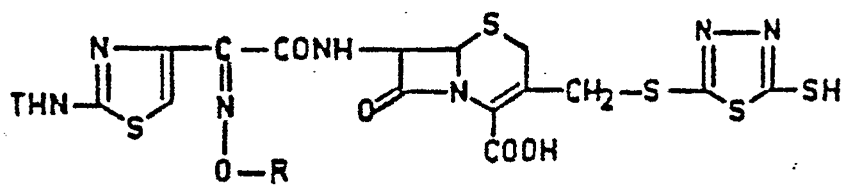

(I')

wherein R and T are as defined above; and, if desired, converting a thus-obtained compound of the formula (I) into a pharmaceutically acceptable salt or ester.

Processes a), b), and c), further the preparation of the protected derivatives of the intermediates used in these processes, the removal of the protecting groups and finally the conversion of the products into pharmaceutically acceptable salts and esters are all very well known methods. The respective patent literature is extremely voluminous; therefore only as examples the US patent specification No. 4,421,912, and European patent publication No. 0,148,004 and the German "Offenlegungsschrift" Nr. 28,04,040 are referred to.

Process a) can be preferably carried out as follows.

To prepare the starting material, 7-ACA of the formula

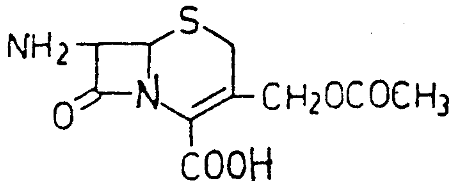

(II)

is reacted with 2,5-dimercapto-1,3,4-thiadiazol of the formula

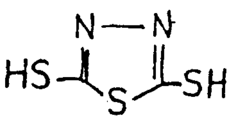

(III)

in acetonitrile. The thus-obtained 7-amino-3-[(2-marcapto-1,3,4-thiadizaol-5-yl)thiomethyl]-ceph-3-em-4-carboxylic acid of the formula (IV) is converted into the corresponding silyl ether by treating it with bis (trimethylsilyl)urea.

The thus-obtained protected acid of the formula (IV) is reacted with a pre-prepared 2-(2-tritylamino-thiazol-4-yl)-(Z)-2-alkoxyiminoacetyl chloride of the formula

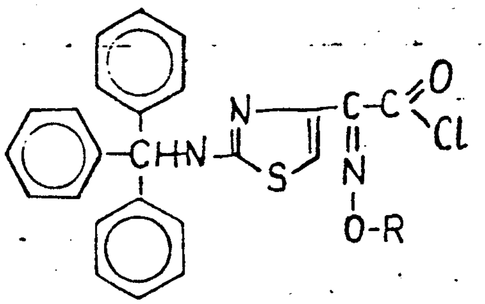

(Va)

wherein R is as defined above, under cooling. The trityl group of the thus-obtained 7β-[2-(2-tritylaminothiazol-4-yl)-(Z)-2-alkoxyimino-acetamido]-3[(2-mercapto-1,3,4-thiadiazol-5-yl)thiomethyl]-ceph-3-em-4-carboxylic acid of the formula

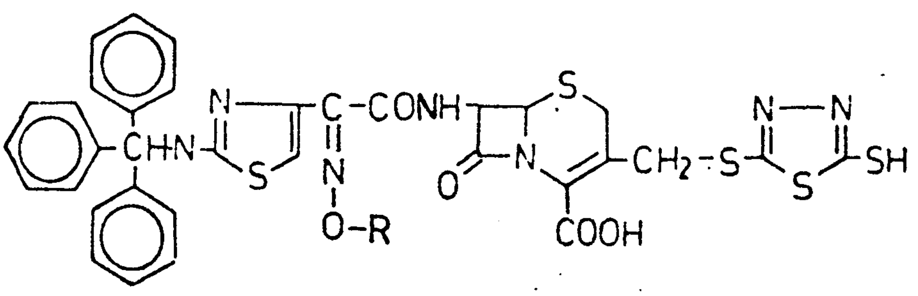

(VI)

is removed by treating it with formic acid and the product of the formula (I) is recovered in a manner known per se.

The carboxylic acids of the formula (I) can be converted into salts, e.g. alkali metal or earth alkali metal salts, or esters, e.g. acetoxymethyl or pivaloyloxymethyl esters, by methods well known in the art, for example by the methods disclosed in the European patent publication No. 0,113,243.

The antibiotic compounds according to the invention may be formulated for administration in any convenient way for use in human or veterinary medicine, according to techniques and procedures per se known in the art with reference to other antibiotics, and the invention therefore includes within its scope a pharmaceutical composition comprising an antibiotic compound according to the present invention such as, for example a pharmaceutically acceptable compound of formula (I) or a salt or in vivo hydrolysable ester thereof together with a pharmaceutically acceptable carrier or excipient.

The compositions may be formulated for administration by any suitable route, such as oral or parenteral, or by topical application. The compositions may be in the form of tablets, capsules, powders, granules, lozenges,

creams or liquid preparations, such as oral or sterile parenteral solutions or suspensions.

Tablets and capsules for oral administration may be in unit dose presentation form, and may contain conventional excipients such as binding agents, for example syrup, acacia, gelatin, sorbitol, tragacanth, or polyvinylpyrollidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, methyl cellulose, glycose syrup, gelatin, hydroxyethylcellulose, carboxymethyl cellulose, aluminium stearate gel or hydrogenated edible fats; emulsifying agents, for example lecithin, sorbitan monooleate, or acacia; non-aqueous vehicles which may include edible oils, for example almond oil, oily esters such as glycerine, propylene glycol, or ethyl alcohol; preservatives, for example methyl or propyl p-hydroxybenzoate or sorbic acid, and if desired, conventional flavouring or colouring agents.

Suppositories will contain conventional suppository base, e.g. cocoa-butter or other glyceride.

For parenteral administration, fluid unit dosage forms are prepared utilising the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing. Advantageously, agents such as local anaesthetic, preservative and buffering agents can be dissolved in the vehicle. To enhance the stability, the composition can be frozen after filling into the vial and the water removed under vacuum. The dry lyophilised powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilisation cannot be accomplished by filtration. The compound can be sterilised by exposure to ethylene oxide before suspending in the sterile vehicle. Advantageously, a surfactant or wetting agent is included in the composition to facilitate uniform distribution of the compound.

The composition may contain from 0.1% to 99.5% by weight, preferably from 10 - 60 % by weight, of the active material, depending on the method of administration. Where the compositions comprise dosage units, each unit will preferably contain from 50-500 mg. of the active ingredient. The dosage as employed for adult human treatment will preferably range from 100 mg. to 12 g. per day for an average adult patient (70 kg.), for instance 1500 mg. per day, depending on the route and frequency of administration. Such dosages correspond to approximately 1.5 to 170 mg./kg. per day. Suitably the dosage is from 1 to 6 g. per day.

The daily dosage is suitably given by administrating a compound of the invention several times in a 24-hour period. Typically, 250 mg. is administered 4 times a day although, in practice, the dosage and frequency of administration which will be most suitable for an individual patient will vary with the age, weight and response of the patients, and there will be occasions when the physician will choose a higher or lower dosage and a different frequency of administration. Such dosage regimens are within the scope of this invention.

No toxicological effects are indicated when a pharmaceutically acceptable compound of the invention of formula (I) or a salt or an ester thereof is administered in the above mentioned dosage range.

The present invention also includes a method of treating bacterial infections in humans and animals which comprises the administration of the therapeutically effective amount of a pharmaceutically acceptable antibiotic compound of this invention of the formula (I) or a salt or an ester thereof.

The following Examples illustrate the preparation of the compounds of the present invention, without limiting, however, the scope of the invention.

Example 1

Preparation of 7β-[2-(2-aminothiazol-4-yl)-(Z)-2-methoxyimino-acetamido]-3-[(2-mercapto-1,3,4-thiadiazol-5-yl)thiomethyl]-ceph-3-em-4-carboxylic acid

(a) Activation of 2-(2-tritylamino-thiazol-4-yl)-(Z)-2-methoxyiminoacetic acid with Vilsmeyer reagent

At a temperature between -5 °C and 0 °C 1.8 ml. (19.7 millimoles) of phosphorus oxychloride are added to 1.5 ml. (19.7 millimoles) of dry dimethyl formamide under constant stirring. The thus-obtained mixture is held at 40 °C for 30 minutes, then cooled to 20 °C and 60 ml. of ethyl acetate are added thereto. At a temperature between -5 °C and 0 °C 7.32 g. (16.47 millimoles) of 2-(2-tritylamino-thiazol-4-yl)-(Z)-2-methoxyiminoacetic acid are added in small portions to said mixture under constant stirring. After the addition the reaction mixture is stirred at the same temperature for 30 minutes.

(b) Silylation of 7β-amino-3-[(2-mercapto-1,3,4-thiadiazol-5-yl)thiomethyl]-ceph-3-em-4-carboxylic acid

5.1 g. (14 millimoles) of 7β-amino-3-[(2-mercapto-1,3,4-thiadiazol-5-yl)thiomethyl]-ceph-3-em-4-carboxylic acid and 19.8 g. (150 millimoles) of monosilyl acetamide are suspended in 180 ml. of ethyl acetate. The suspension is boiled in nitrogen atmosphere under constant stirring for 30 minutes.

(c) Acylation

The solution obtained in step (b) above is cooled to -20 °C and in nitrogen atmosphere the solution obtained in step (a) above is added under constant stirring thereto during 15-20 minutes. After the addition the reaction mixture is let to warm to -10 °C and stirred at this temperature for 2 hours. After the termination of the reaction the reaction mixture is added slowly to a mixture of 210 ml. of saturated potassium chloride solution and 420 ml. of ethyl acetate cooled to -20 °C. The insoluble precipitate is removed on a perlite bed, the phases are separated and the aqueous phase is extracted twice with 100 ml. of ethyl acetate each. The combined organic phases are extracted twice with 100 ml. of saturated potassium chloride solution each, dried over magnesium sulfate and evaporated to dryness under reduced pressure. The residue is triturated with ether, filtered, washed with ether and finally dried first with air, then over phosphorus pentoxide in vacuo.

Yield: 8 g.

(d) 7β-[2-(2-Aminothiazol-4-yl)-(Z)-2-methoxyiminoacetamido]-3-[(2-mercapto-1,3,4-thiadiazol-5-yl) thiomethyl]-ceph-3-em-4-carboxylic acid

0.6 g of 7β-[2-(2-tritylamino-thiazol-4-yl)-(Z)-2-methoxyimino-acetylamino]-3-[(2-mercapto-1,3,4-thiadiazol-5-yl)-thiomethyl]-ceph-3-em-4-carboxylic acid are dissolved in 10 ml. of formic acid of 85 % strength. The solution is stirred at room temperature for 2 hours. After adding 5 ml. of water the precipitated triphenyl carbinol is filtered off and washed with a small amount of water. The filtrate is evaporated to dryness at 40-45 °C, the residue is triturated with ether, filtered off and washed with ether, finally it is dried first with air, then over phosphorus pentoxide in vacuo.

Yield: 0.4 g

$^1$H NMR (ppm) DMSO-$d_6$: 3.67 (ABq, 2H, H-2), 3.95 (s, 3H, $OCH_3$), 4.23 (ABq, 2H, H-10), 5.2 (d, 1H, H-6), 5.8 (q, 1H, H-7), 6.9 (s, 1H, s, H).

Example 2

Preparation of
7β-[2-(2-aminothiazol-4-yl)-(Z)-2-methoxyimino-acetamido]-3-[(2-mercapto-1,3,4-thiadiazol-5-yl)thiomethyl]-ceph-3-em-4-carboxylic acid

(a) 7-Amino-3-[(2-mercapto-1,3,4-thiadiazol-5-yl) thiomethyl]-ceph-3-em-4-carboxylic acid

20 g. (7.34 millimoles) of 7-ACA and 12 g. (7.98 millimoles) of 2,5-dimercapto-1,3,4-thiadiazole are suspended in 150 ml. of dry acetonitrile. The suspension is cooled to -5 °C under constant stirring. At this temperature 30 ml. of chlorosulfonic acid are added so that the inner temperature should not exceed -5 °C. After terminating the addition the reaction mixture is let to warm to 10 °C and stirred at this temperature for 40 minutes. After the termination of the reaction the reaction mixture is added dropwise to 500 ml. of water of 0 °C under constant cooling and stirring so that the inner temperature should not exceed 0 °C. After terminating the addition the pH of the so-obtained solution is set to 3.8 by using first 10 N, then 5 N sodium hydroxide solution at 0 °C under cooling and stirring. The precipitated material is let to sediment at 0 °C for two hours and then recovered by filtration. It is washed consecutively with water of a pH of 3.8, methanol and acetone, finally it is dried over phosphorous pentoxide in vacuo.

(b)
7β-[2-(2-Tritylamino-thiazol-4-yl)-(Z)-methoxyimino-acetamido]-3-[(2-mercapto-1,3,4,-thiadiazol-5-yl)thiomethyl]-ceph-3-em-4-carboxylic acid

(i) Preparation of acid chloride from 2-(2-tritylamino-thiazol-4-yl)-(Z)-2-methoxyiminoacetic acid

1.32 g. (3 millimoles) of 2-(2-tritylamino-thiazol-4-yl)-(Z)-2-methoxyiminoacetic acid and 0.42 ml. (3 millimoles) of triethyl amine are dissolved in 24 ml. of dry dichloromethane. The solution is cooled to a temperature between -5 °C and -10 °C and at this temperature a solution of 0.66 g. (3 millimoles) of phosphorus pentachloride in 10 ml. of dry dichloromethane is added thereto dropwise under constant stirr ing. After the addition the reaction mixture is stirred at a temperature between -5 °C and -10 °C for 10 minutes. The dichloromethane is evaporated. The residue is thoroughly triturated with dry benzene, then the benzene is evaporated. The residue is thoroughly triturated with dry petrol ether, decanted and the rest of the petrol ether is removed by evaporation. The thus-obtained solid material is dissolved in acetonitrile. This solution is used for acylating.

(ii) Silylation of 7β-amino-3-[(2-mercapto-1,3,4-thiadiazol-5-yl)thiomethyl]-ceph-3-em-4-carboxylic acid

0.72 g. (2 millimoles) of 7β-amino-3-[(2-mercapto-1,3,4-thiadiazol-5-yl) thiomethyl]-ceph-3-em-4-carboxylic acid and 1.2 g (6.0 millimoles) of bis(trimethylsilyl) urea are suspended in 30 ml. of dry acetonitrile. The suspension is heated to its boiling point under stirring and nitrogen atmosphere and then boiled for 40 minutes.

(iii) Acylation

The solution obtained in step (ii) above is cooled to -10 °C. At this temperature the solution of the acid chloride obtained in step (i) above is added dropwise to this solution under nitrogen atmosphere. After the addition the reaction mixture is let to warm to room temperature and stirred at this temperature for 2 hours. After the termination of the reaction the acetonitrile is removed by evaporation. The residue is taken up with a mixture of water and ethyl acetate at 0 °C, the insolubles are removed by filtration and the phases are separated. The aqueous phase is extracted with ethyl acetate. The combined organic phases are dried over magnesium sulfate and evaporated, the residue is triturated with ether, filtered and dried over phosphorus pentoxide in vacuo.

Yield: 0.60 g.

$^1H$ NMR (ppm) DMSO-$d_6$: 3.60 (ABq, 2H, H-2), 3.80 (s, 3H, $OCH_3$), 4.17 (ABq, 2H, H-10), 5.10 (d, 1H, H-6), 5.7 (q, 1H, H-7), 6.7 (s, 1H, 2, H), 7.12-7.47 (m, 15H, H-Ar), 8.92 (s, 1H, Ar-NH), 9.57 (d, 1H, C-7NH).

(c)

7β-[2-(2-aminothiazol-4-yl)-(Z)-2-methoxyimino-acetamido]-3-[(2-mercapto-1,3,4-thiadiazol-5-yl)thiomethyl]-ceph-3-em-4-carboxylic acid

One proceeds as in step (d) of Example 1. The same product is obtained in the same amount, with an identical NMR-spectrum.

## Claims

1. A 7β-[2-(2-aminothiazol-4-yl)-(Z)-2-alkoxyimino-acetamido]-3-[(2-mercapto-1,3,4-thiadiazol-5--yl)thiomethyl]-ceph-3-em-4-carboxylic acid of the formula

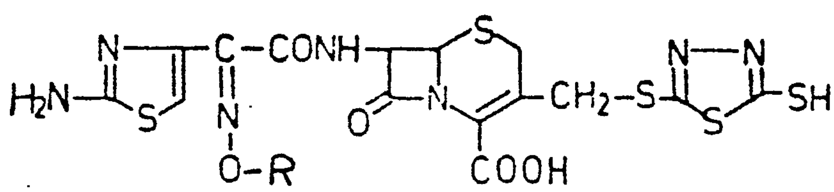

(I),

wherein R represents a $C_{1-4}$ alkyl group, or a pharmaceutically acceptable salt or ester thereof.

2. 7β-[2-(2-aminothiazol-4-yl)-(Z)-2-methoxyimino-acetamido]-3-[(2-mercapto-1,3,4-thiadiazol-5-yl)thiomethyl]-ceph-3-em-4-carboxylic acid or a pharmaceutically acceptable salt or ester thereof.

3. Process for the preparation of a 7β-[2-(2-aminothiazol-4-yl)-(Z)-2-alkoxyimino-acetamido]-3-[(2-mercapto-1,3,4-thiadiazol-5-yl)thiomethyl]-ceph-3-em-4-carboxylic acid of the formula

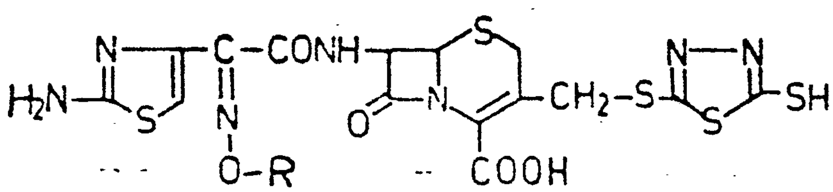

(I),

wherein R represents a $C_{1-4}$ alkyl group, or a pharmaceutically acceptable salt or ester thereof, **characterized** by

(a) acylating a carboxy-protected derivative of the compound of the formula

(IV)

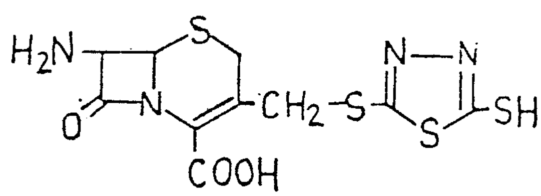

with a compound of the formula

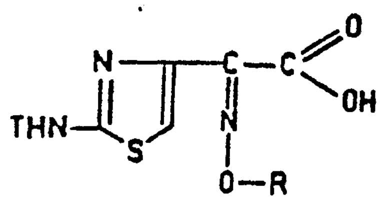

(V),

wherein T is an amino-protecting group and R is as defined above, or a reactive derivative thereof and removing the protecting groups; or

(b) reacting a carboxy-protected derivative of the compound of the formula

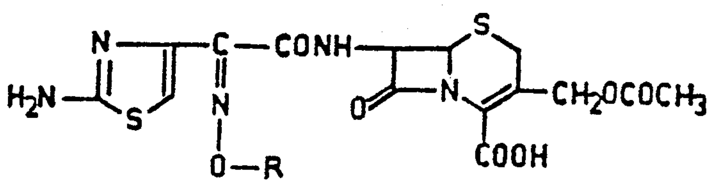

(VII)

with a compound of the formula

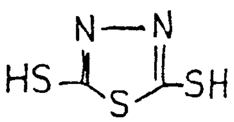

(III),

and removing the protecting group; or

(c) removing the T amino-protecting group and, if present, the carboxy-protecting group of a compound of the formula

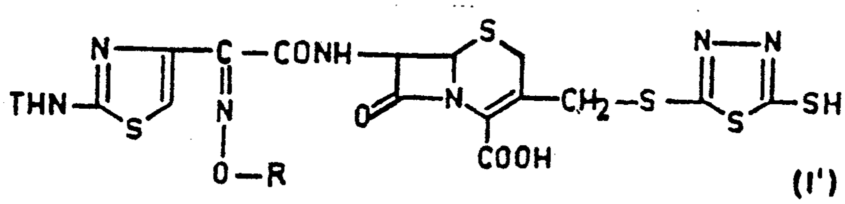

(I')

wherein R and T are as defined above; and, if desired, converting a thus-obtained compound of the formula (I) into a pharmaceutically acceptable salt or ester.

4. A process as claimed in claim 3, **characterized** by preparing 7β-[2-(2-aminothiazol-4-yl)-(Z)-2-methoxyimino-acetamido]-3-[(2-mercapto-1,3,4-thiadiazol-5-yl)thiomethyl]-ceph-3-em-4-carboxylic acid or a pharmaceutically acceptable salt or ester thereof.

5. Pharmaceutical composition, **characterized** by containing a therapeutically effective amount of a compound of the formula

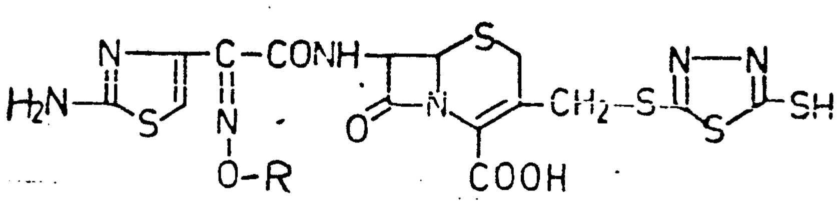

(I),

wherein R represents a $C_{1-4}$ alkyl group, or a pharmaceutically acceptable salt or ester thereof.

6. A composition as claimed in claim 5, **characterized** by containing 7β-[2-(2-aminothiazol-4-yl)-(Z)-2-methoxyimino-acetamido]-3-[(2-mercapto-1,3,4-thiadiazol-5-yl)thiomethyl]-ceph-3-em-4-carboxylic acid or a pharmaceutically acceptable salt or ester thereof.

7. Method of treating bacterial diseases **characterized** by using a therapeutically effective amount of a compound of the formula

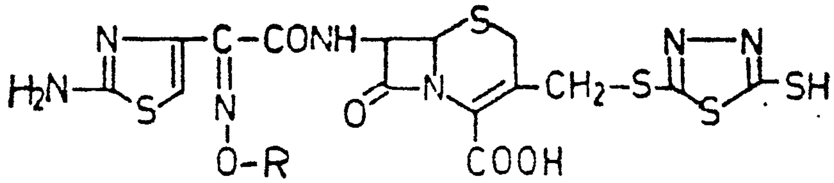

(I),

wherein R represents a $C_{1-4}$ alkyl group, or a pharmaceutically acceptable salt or ester thereof.

8. A method as claimed in claim 7, **characterized** by using 7β-[2-(2-aminothiazol-4-yl)-(Z)-2-methoxyimino-acetamido]-3-[(2-mercapto-1,3,4-thiadiazol-5-yl)thiomethyl]-ceph-3-em-4-carboxylic acid or a pharmaceutically acceptable salt or ester thereof.

9. Use of the compound of the general formula I where R is as defined in claim 1 or a pharmaceutically acceptable salt or ester thereof for use in the manufacture of medicinal compositions for the treatment of bacterial diseases, especially diseases caused by Gram-negative bacteria and particularly diseases caused by Pseudomonas Spp.

CLAIMS For Austria

1. Process for the preparation of a 7β-[2-(2-aminothiazol-4-yl)-(Z)-2-alkoxyimino-acetamido]-3-[(2-mercapto-1,3,4-thiadiazol-5-yl)thiomethyl]-ceph-3-em-4-carboxylic acid of the formula

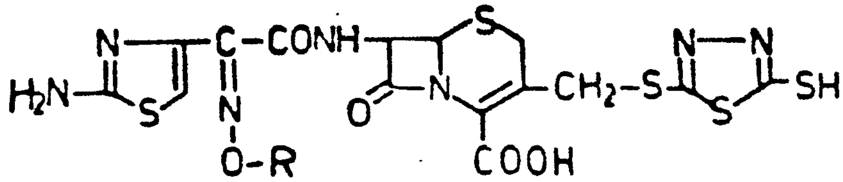

(I),

wherein R represents a $C_{1-4}$ alkyl group. or a pharmaceutically acceptable salt or ester thereof, **characterized** by

(a) acylating a carboxy-protected derivative of the compound of the formula

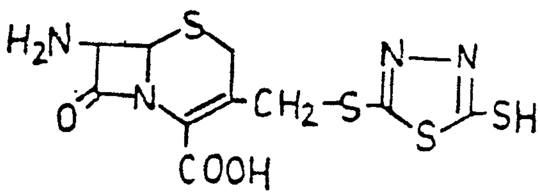

(IV)

with a compound of the formula

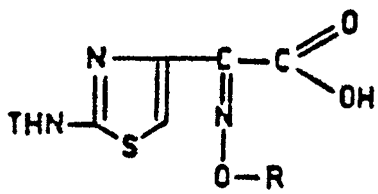

(V),

wherein T is an amino-protecting group and R is as defined above, or a reactive derivative thereof and removing the protecting groups; or

(b) reacting a carboxy-protected derivative of the compound of the formula

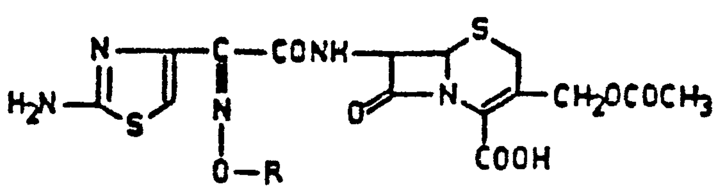

(VII)

with a compound of the formula

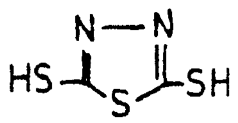

(III),

and removing the protecting group; or

(c) removing the T amino-protecting group and, if present, the carboxy-protecting group of a compound of the formula

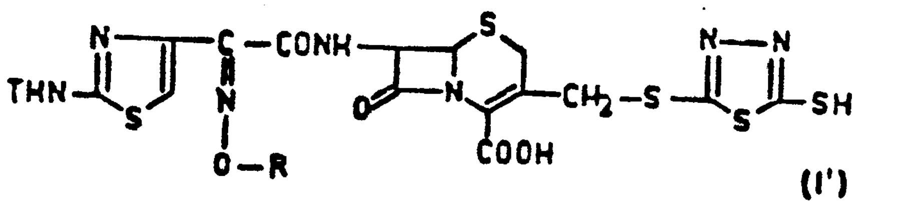

(I')

(I')

wherein R and T are as defined above; and, if desired, converting a thus-obtained compound of the formula (I) into a pharmaceutically acceptable salt or ester.

2. A process as claimed in claim 1, **characterised** by preparing 7β-[2-(2-aminothiazol-4-yl)-(Z)-2-methoxyimino-acetamido]-3-[(2-mercapto-1,3,4-thiadiazol-5-yl)thiomethyl]-ceph-3-em-4-carboxylic acid or a pharmaceutically acceptable salt or ester thereof.

3. A process of preparing a pharmaceutical compositions, which process comprises mixing, dissolving or suspending a therapeutically effective amount of a compound of the formula

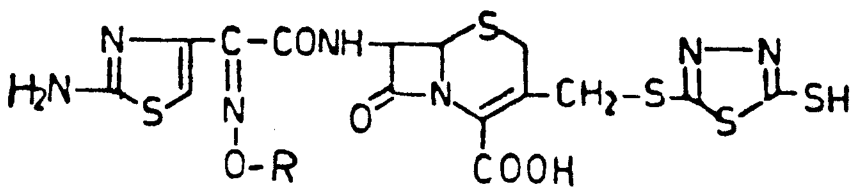

(I),

wherein R represents a $C_{1-4}$ alkyl group and preferably a methyl group, or a pharmaceutically acceptable salt or ester thereof with or in a pharmaceutically acceptable solid or liquid carrier or excipient and optionally forming the thus obtained composition into unit dosage forms.

4. Use of the compound of the general formula I where R is as defined in claim 1 or a pharmaceutically acceptable salt or ester thereof for use in the manufacture of medicinal compositions for the treatment of bacterial diseases, especially diseases caused by Gram-negative bacteria and particularly diseases caused by Pseudomonas Spp.